# EUROPEAN PATENT APPLICATION

(11) **EP 4 484 004 A1**
(43) Date of publication of application: **01.01.2025**
(21) Application number: 22718285.4
(22) Date of filing: 28.02.2022
(51) Int. Cl.: B01J 23/72, B01J 23/80, B01J 21/06, B01J 35/10, B01J 37/02, B01J 37/34, C02F 1/72, C02F 1/50, C02F 1/32, B01J 37/08, A61L 9/20, B01J 37/00, B01J 23/00

(54) **AIR FILTER WITH PHOTOCATALYTIC COATING AND MANUFACTURING METHOD THEREOF**

(30) Priority: 25.02.2022 PT 2022117817
(71) Applicant: Vieira & Lopes Lda, 4730-460 Prado (PT)
(72) Inventor: SOUSA, Juliana, 4515-114 Foz-do-sousa (PT); LIMA DE SOUSA, Viviana, 4720-139 Amares (PT); DE MENDONÇA SPERA, Natalia Cristina, 4730-241 Vila Verde (PT)
(74) Representative: Patentree
(86) International application number: PCT/IB2022/051738
(87) International publication number: WO 2023/161686

(57) **Abstract**

The present description concerns an air filter with photocatalytic coating for the inactivation of viruses and manufacturing method thereof, comprising a calcined mixture of copper with titanium dioxide or with zinc oxide nanoparticles, wherein the amount of copper is 0.3 - 30 % (wt/wt) relatively to the total weight of the coating.

## Description

### TECHNICAL FIELD

The present description relates to an air filter with photocatalytic coating for the inactivation of viruses and to the obtaining method thereof. More specifically, it refers to an air filter with photocatalytic coating which comprises a calcined mixture of copper with titanium dioxide nanoparticles or copper with zinc oxide nanoparticles.

### BACKGROUND

The document WO2018110173A1 discloses a photocatalytic material which comprises a base and a photocatalytic layer disposed on one surface of the base. The photocatalytic layer comprises photocatalyst particles, copper compound particles comprising copper(II) oxide and/or copper(II) hydroxide, inorganic particles having no photocatalytic activity and an inorganic binder, the amount of copper contained in the copper compound particles being 0.1-5 parts by weight per 100 parts by weight of the photocatalyst particles. The copper compound particles have been fixed so as to be in contact with the surfaces of the photocatalyst particles and the inorganic particles. It further describes a photocatalytic coating composition which is for forming the photocatalytic layer of said photocatalytic material. The photocatalytic coating composition comprises photocatalyst particles, a copper compound which is a precursor of copper(II) oxide and copper(II) hydroxide and which comprises a compound of a divalent copper ion, inorganic particles having no photocatalytic activity and a precursor of an inorganic binder, the amount of the copper contained in the copper compound being 0.1-5 parts by weight per 100 parts by weight of the photocatalyst particles.

The document WO2011078203A1 discloses a virus inactivator that can exhibit an inactivation activity involving structural disruption such as denaturation or decomposition on viruses and which comprises a univalent copper compound such as copper oxide, copper sulphide, copper iodide and copper chloride as an active ingredient, and a virus-inactivating material which comprises a base material and the virus inactivator on the surface and/or inside the base material. The document WO2011078203A1 does not present a solution with high stability since the copper can be released to the environment.

These facts are described in order to illustrate the technical problem solved by the embodiments of this document.

### GENERAL DESCRIPTION

The present description refers to an air filter with photocatalytic coating for the inactivation of viruses, comprising a calcined mixture of copper nanoparticles with titanium dioxide or zinc oxide, wherein the amount of copper is 0.3 - 30% (wt/wt) relatively to the coating.

In an embodiment, the photocatalytic coating comprises a mixture of copper with titanium dioxide, wherein the amount of copper is 0.3 - 30% (wt/wt) relatively to the coating.

In an embodiment, the photocatalytic coating comprises a mixture of copper with zinc oxide, wherein the amount of copper is 0.3 - 30% (wt/wt) relatively to the coating.

In an embodiment, the calcination temperature is 100 - 400°C, more particularly 100 - 200°C, even more particularly approximately 100°C.

In an embodiment, the amount of copper is 0.5 -15% (wt/wt).

In an embodiment, the amount of copper is 0.5%; 1%; 3%; 6%; 10%; 12% or 15% (wt/wt).

In an embodiment, the copper with titanium dioxide or with zinc oxide nanoparticles are obtainable by wet impregnation.

In an embodiment, the copper with titanium dioxide or with zinc oxide nanoparticles are obtainable by incipient impregnation.

In an embodiment, the air filter has a metal mesh, in particular an aluminium metal mesh.

The present description also concerns a manufacturing method of the air filter of any of the preceding claims, which comprises the steps of:
providing a metal filter mesh;
obtaining copper nanoparticles impregnated with titanium dioxide or with zinc oxide;
obtaining a suspension by mixing the titanium dioxide or zinc oxide copper nanoparticles with silane;
coating the metal mesh with the suspension obtained in the previous step;
curing at 80°C for a period of 24 hours.

In an embodiment, obtaining the nanoparticles, the copper is impregnated in the titanium dioxide or zinc oxide by wet impregnation or incipient impregnation.

In an embodiment, the obtention of the nanoparticles comprises a step of thermal treatment at 100 - 400°C.

In an embodiment, the amount of silane in the suspension is of 90% (v/v).

In an embodiment, the metal mesh is coated by spray coating.

### BRIEF DESCRIPTION OF THE DRAWINGS

For an easier understanding, the figures are herein attached, which represent preferred embodiments which are not intended to limit the object of the present description.
**Figure 1****:** Graphic representation of the XRD related to an embodiment with CuO impregnation in TiO₂ and calcined at 100 °C by the incipient impregnation method (a) and wet impregnation method (b). The data lines are ordered in the same order as they are represented in the legend, representing the relative intensity (u.a.) and the grade.
**Figure 2****:** Graphic representation of the XRD related to CuO impregnated in TiO₂ and calcined at 200 °C by the incipient impregnation method (a) and wet impregnation method (b).
**Figure 3****:** Graphic representation of the XRD related to CuO impregnated in TiO₂ and calcined at 400 °C by the incipient impregnation method (a) and wet impregnation method (b).
**Figure 4****:** Graphic representation of the XRD related to CuO impregnated in ZnO by incipient impregnation and calcined at 100°C (a) and at 200 °C (b).
**Figure 5****:** Graphic representation of the XRD related to CuO impregnated in ZnO by wet impregnation and calcined at 200 °C (a) and at 400 °C (b).
**Figure 6****:** Photographic representation of an embodiment of the coated filter #1 CuO/ZnO 1% W 200°C with lower magnification (a) and higher magnification (b).
**Figure 7****:** Photographic representation of an embodiment of the coated filter #2 CuO/TiO₂ 15% I 200°C with lower magnification (a) and higher magnification (b).
**Figure 8****:** Photographic representation of an embodiment of the coated filter #3 CuO/TiO₂ 1% W 100°C with lower magnification (a) and higher magnification (b).
**Figure 9****:** Photographic representation of an embodiment of the coated filter #4 CuO/TiO₂ 0.5% W 100°C with lower magnification (a) and higher magnification (b).
**Figure 10****:** Photographic representation of an embodiment of the coated filter #5 CuO/ZnO 6% W 200°C with lower magnification (a) and higher magnification (b).
**Figure 11****:** Photographic representation of an embodiment of the coated filter #6 CuO/ZnO 6% W 400°C with lower magnification (a) and higher magnification (b).
**Figure 12****:** Photographic representation of an embodiment of the coated filter #7 CuO/ZnO 15% W 200°C with lower magnification (a) and higher magnification (b).
**Figure 13****:** Photographic representation of an embodiment of the coated filter #8 CuO/TiO₂ 6% I 200°C with lower magnification (a) and higher magnification (b).

### DETAILED DESCRIPTION

Coronaviruses are a class of enveloped viruses protected by a lipid bilayer membrane, where the new SARS-COV-2 virus is included. The photocatalytic activity of titanium dioxide and zinc oxide is able to cause modifications on the virus proteins. These modifications decrease the virus' ability to bind to its host's receptors and inactivate its infectious activity.

Unlike other air purifiers that capture only particles, photocatalysis is a promising technology as it is able to convert indoor air pollutants into non-harmful products. Titanium dioxide (TiO₂) and zinc oxide (ZnO) are quite promising photocatalysts due to their non-toxicity, low cost, high stability and high reactivity. In order to increase the antiviral properties of these materials, nanocomposites were prepared, preferably nanoparticles with copper. Copper is known for its antimicrobial properties, being able to damage viral genomic DNA. Regarding this, nanocomposites of copper, with titanium dioxide or zinc oxide were prepared in order to create a synergetic effect able to degrade SARS-COV-2 virus. The effective inactivation of the virus will result from the attack of the copper ions and the reactive oxygen species formed during the photocatalysis process.

In an embodiment, the photocatalytic materials are synthetized in powder form.

In an embodiment, this material will be applied in air purifiers, it being necessary to incorporate it into the air filters in a way that the filter can be reused for a long period of time. The photocatalytic nanoparticles allow that, by photocatalysis, the filter is always free from viruses or pollutants. During this work an inorganic coating was developed, so that the nanocomposites produced continue to exhibit a high catalytic activity and present good adhesion properties, in order to guarantee that the photocatalytic materials are not released into the environment.

For the embodiments of the present description, the photocatalytic material, zinc oxide and titanium dioxide (TiO₂ and ZnO), was impregnated with copper nitrate by the method of wet impregnation (W) or by the method of incipient impregnation (I). The zinc oxide and titanium oxide materials were impregnated with the following amounts of copper (CuO): 0.5, 1, 6, 15 and 25 % (wt/wt). The samples were thermal treated at 100, 200 and 400 °C for 4 hours. The prepared materials were characterized by XRD.

Preferably, wet impregnation consists of incorporating a metal precursor in a volume equal to the pore volume of the photocatalytic material. The pore volume of the photocatalytic material, namely zinc oxide or titanium dioxide, ranges from 0.5 to 3 cm³/g, preferably 1 cm³/g and the pore size from 1 to 5 nm, preferably 2 nm. The measurement of porosity has been carried out by nitrogen adsorption isotherms at -196 °C. Wet impregnation is performed as follows: in a first stage, the metal precursor - copper nitrate - is dissolved in 1 mL of water. Adding 1g of titanium dioxide or zinc oxide in an ultrasonic bath to improve the dispersion of the metal and subsequently, adding the precursor solution to the material, drop by drop. The samples stay at ultrasonic bath for 90 minutes and are then placed in an oven to dry at 110 °C for 12 hours.

Preferably, in the incipient impregnation, the volume of the metal precursor is in excess compared to the pore volume of the photocatalytic material. The pore volume of the photocatalytic material, namely zinc oxide or titanium dioxide, ranges from 0.5 to 3 cm³/g, preferably 1 cm³/g, and the pore size ranges from 1 to 5 nm, preferably 2 nm. The measurement of porosity has been carried out by nitrogen adsorption isotherms at -196 °C. Incipient impregnation is performed as follows: placing 1g of titanium dioxide or zinc oxide under magnetic stirring with the aqueous solution of the metal precursor for 24 hours. Subsequently, the mixture is filtered and dried in an oven at 110 °C for 12 hours.

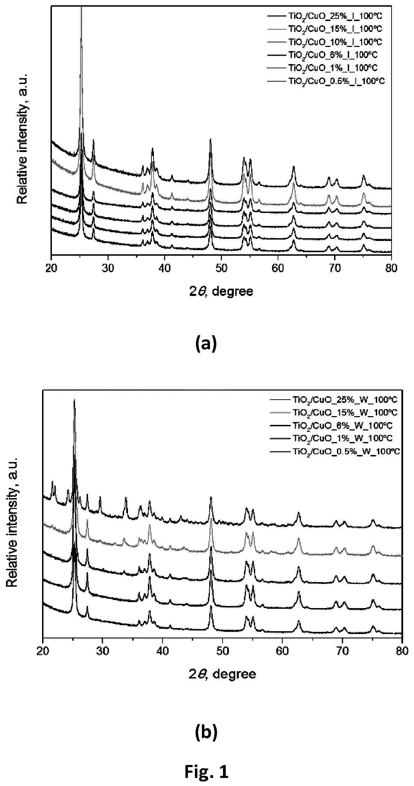

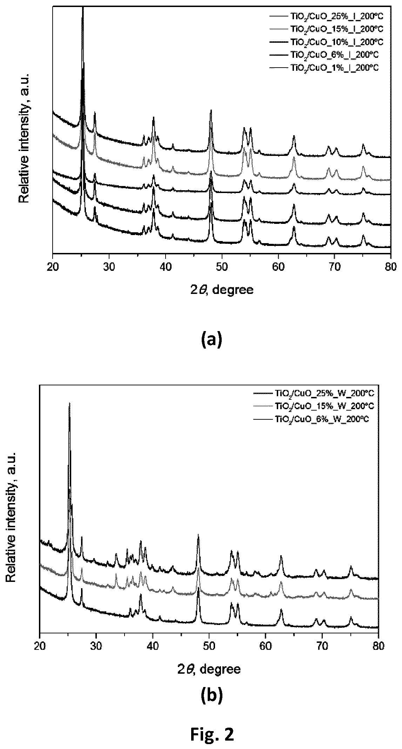

Photocatalytic materials of TiO₂ after impregnation with different concentrations of CuO, prepared at different calcination temperatures, were characterized by XRD, as can be seen in Figures 1 to 3.

For those materials impregnated by the incipient impregnation method no peaks related to the presence of CuO were detected at any calcination temperature (Figures 1 to 3). Only rutile and anatase phases correspondent to TiO₂ were confirmed. Relatively to the wet impregnation method, the presence of TiO₂ phases was confirmed and the presence of CuO was verified for concentrations of 15 and 25% at calcination temperatures of 200 and 400 °C. At the temperatur of 100 °C, for the concentrations of 6, 15 and 25% other phases relative to TiO₂ hexagonal appear. For the concentration of 25%, other secondary phases of TiO₂ appeared in addition to the typical peaks of Cu₂O and CuO.

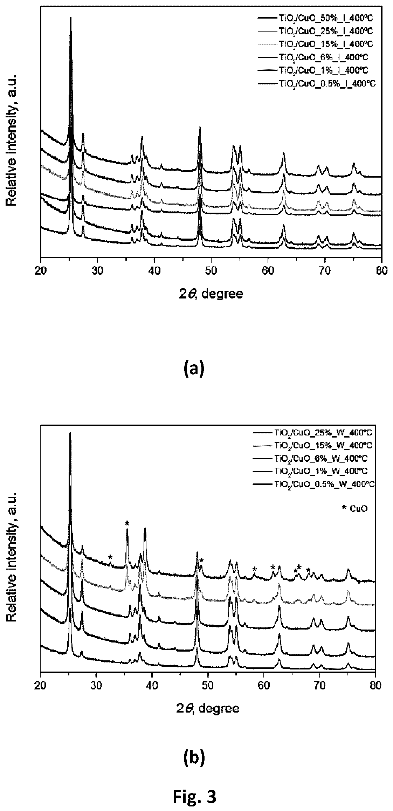

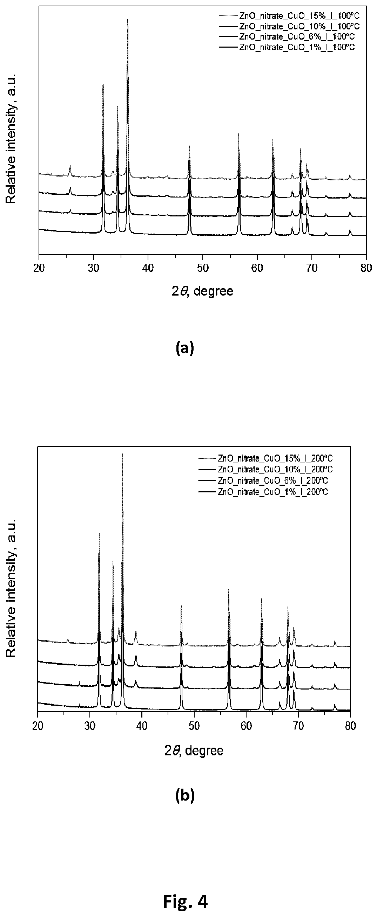

Photocatalytic materials of ZnO after impregnation with different concentrations of CuO, prepared at different calcination temperatures were characterized by XRD, as can be seen in Figures 3 and 4.

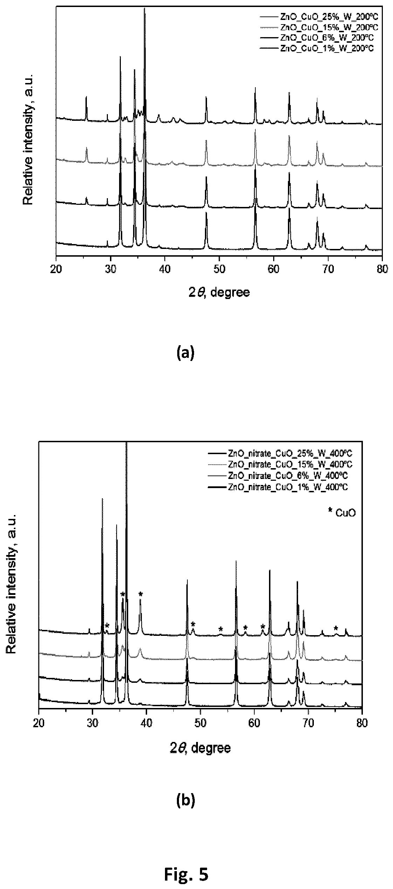

For those materials impregnated by the incipient impregnation method and calcinated at 100 °C, only the chrystalline phase of ZnO is verified, whereas for the concentrations of CuO of 6, 10 and 15% (w/w) also the presence of CuZn phases is observed (Figure 4). For those materials calcinated at 200 °C it is confirmed the presence of CuO peaks for all the concentrations (Figure 4). Relatively to the wet impregnation method, both at 200 °C and 400 °C it is confirmed the presence of CuO peaks for all the concentrations (Figure 5). It should be noted that for the CuO concentration of 25 % at 200 °C, peaks related to Cu and CuN phases also appear (Figure 5).

The photocatalytic activity of the prepared samples was tested by using a standard molecule, methylene blue. The aqueous solution of methylene blue used had a concentration of 9 mg·L⁻¹. 5 mg of photocatalytic material was added in 50 mLof methylene blue solution. Prior to the experiments with the presence of UV radiation, it was checked whether the materials presented adsorption properties, and for that, the material was under stirring in the dark for 2 hours. It was found that none of the samples prepared showed adsorption properties. Thus, the concentration reduction of the dye over time is only due to the catalytic activity of the materials. Right after the adsorption experiments, the UV light was turned ON, and samples have been taken over time, and the concentration of the samples was analysed by UV-VIS Spectroscope. The obtained results for the titanium dioxide samples with different percentages of copper prepared by wet impregnation and calcined at different temperatures are shown in Table 1.

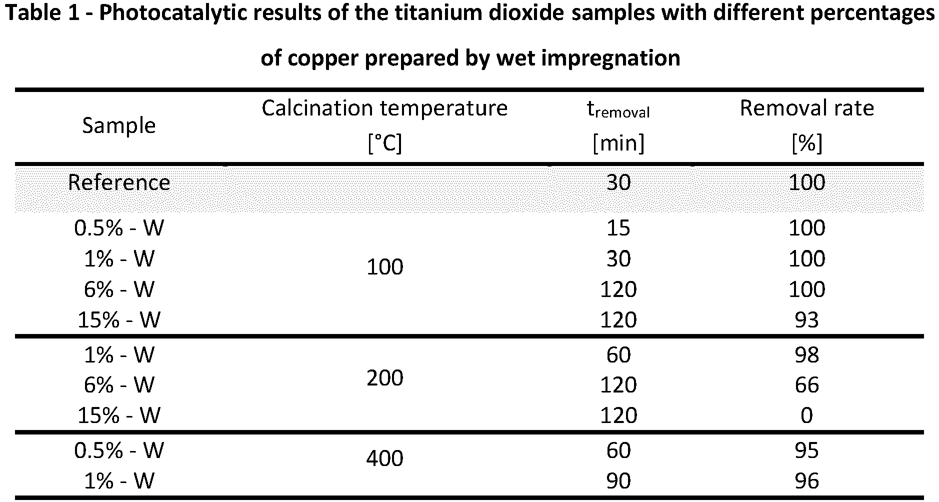

The reference sample, which corresponds to the titanium dioxide without the presence of copper, takes only 30 minutes to completely degrade the dye. It was observed that the incorporation of 0.5% of copper increases the photocatalytic activity of the titanium dioxide and the sample is able to fully degrade the dye in just 15 minutes. By increasing the concentration of copper, higher than 1 %, the materials begin to lose their photocatalytic activity, with the sample prepared with 15 % of copper and calcined at 200 °C not presenting any reduction of dye concentration even after 2 hours of reaction.

The increase of the calcination temperature also has a great influence on the photocatalytic activity of the materials; the sample with 0.5 % of copper, calcined at 400 °C needs 60 minutes to degrade the dye, while the sample calcined at 100 °C only needed 15 minutes. This result can be explained by the conversion of anatase into rutile by increasing the calcination temperature.

The results obtained for the titanium dioxide samples with different percentages of copper by incipient impregnation and calcined at different temperatures are shown in Table 2.

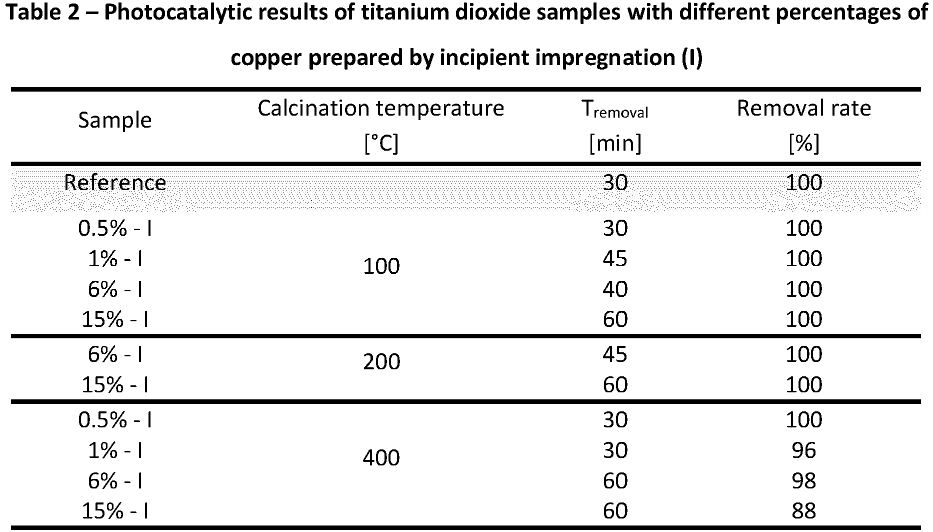

Comparing the results from Table 1 and 2, the samples prepared by incipient impregnation were found to exhibit a more uniform behaviour with each other when compared to the behaviour presented by the samples prepared by wet impregnation. Unlike to what was previously observed, even with 15% copper, the samples present photocatalytic activity and are able to degrade the dye. The sample prepared with 15% of copper and calcined at 200 °C is able to completely degrade the dye after 60 minutes. By increasing the calcination temperature to 400 °C, the sample is able to degrade 88% of the initial concentration of dye after one hour.

The photocatalytic results obtained for the zinc oxide samples with different percentages of copper prepared by wet impregnation and calcined at different temperatures are shown in Table 3.

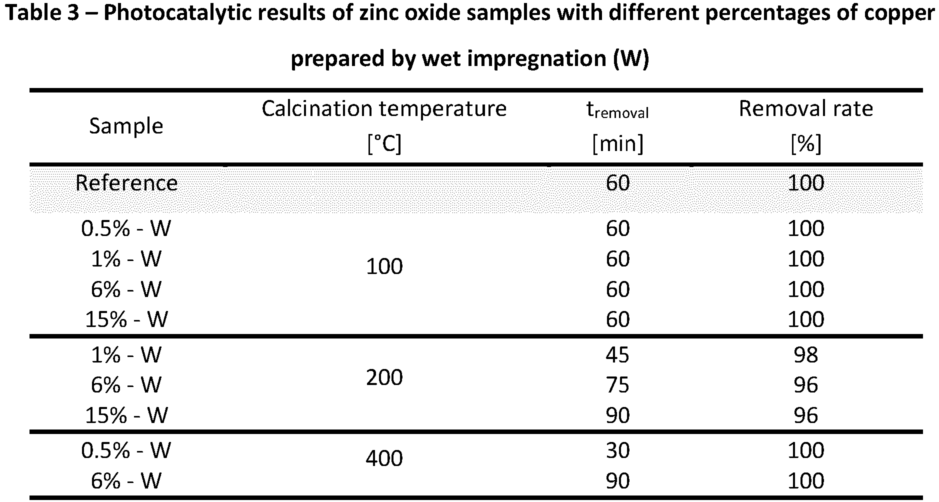

The reference sample, zinc oxide without the presence of copper, needs 60 minutes to completely degrade the dye. The zinc oxide needs more time than the titanium dioxide to degrade the dye. However, comparing the results shown in this table with the ones shown in Table 2, it was found that the presence of a great amount of copper does not eliminate the photocatalytic activity of the material, as has been observed for the titanium dioxide. It can be concluded that using zinc oxide it is possible to prepare a photocatalytic material with a high concentration of copper, up to 15%. Furthermore, it was found that using titanium dioxide the increase of the calcination temperature worsened its photocatalytic activity, with the zinc oxide, it was observed that the temperature does not have a significant influence on the catalytic activity of the material. It was found that, in some cases, the use of a higher calcination temperature can even improve the photocatalytic activity of the materials. The sample prepared with 0.5% of copper and calcined at 400 °C needs only 30 minutes to degrade the dye, showing a superior photocatalytic activity compared to the reference material.

The photocatalytic coating was prepared for a 100 mL solution. It started by mixing 26 mL of ethanol, 15.5 mL of tetraethyl orthosilicate (TEOS), 35.8 mL of 3-glycidoxypropyl trimethoxysilane (GPTMS), 13.8 mL of deionized water with pH 1 (hydrochloric acid was used to adjust the pH). Then, 25 mL of ethanol with 0.6 g of 3-(Trimethoxysilyl)propyldimethyl-octadecyl ammonium chloride (HM4100) or 25 mL and 10 g of the corresponding precursor, preferable copper with titanium dioxide or zinc oxide nanoparticles were added to the previous solution of silanes and left under vigorous stirring until a uniform dispersion was ensured. To improve the dispersion of the particles, they were subjected to 5000 RPM for 1 hour in DISPERMAT^{®} equipment.

The silane coating with the photocatalytic material was applied by spray technique. Three layers of coating were applied to the filters with dimensions of 30 x 30 cm. Each selected coating was applied to 4 filters, only on one side. Between each application, the filters were placed inside the oven at 80 °C for 1 minute in order to evaporate the solvent. After the three spray applications, the coating was subjected to a curing process at 80 °C for 24 hours. In Table 4, it is shown the list and respective description of the different coatings applied to the filters/final prototypes.

**Table 4 - Description of the samples prepared with the best selected coatings and respective description of reagents and application process.**

| # | Code | Reagents |
|---|---|---|
| 1 | ZnO/CuO 1% W 200°C | ZnO NPs + copper nitrate |
| 2 | TiO₂/CuO 15% I 200°C | TiO₂ NPs + copper nitrate |
| 3 | TiO₂/CuO 1% W 100°C | TiO₂ NPs + copper nitrate |
| 4 | TiO₂/CuO 0.5% W 100°C | TiO₂ NPs + copper nitrate |
| 5 | ZnO/CuO 6% W 200°C | ZnO NPs + copper nitrate |
| 6 | ZnO/CuO 6% W400°C | ZnO NPs + copper nitrate |
| 7 | ZnO/CuO 15% W 200°C | ZnO NPs + copper nitrate |
| 8 | TiO₂/CuO 6% I 200°C | TiO₂ NPs + copper nitrate |

Where NPs refers to nanoparticles, W wet impregnation and I incipient impregnation.

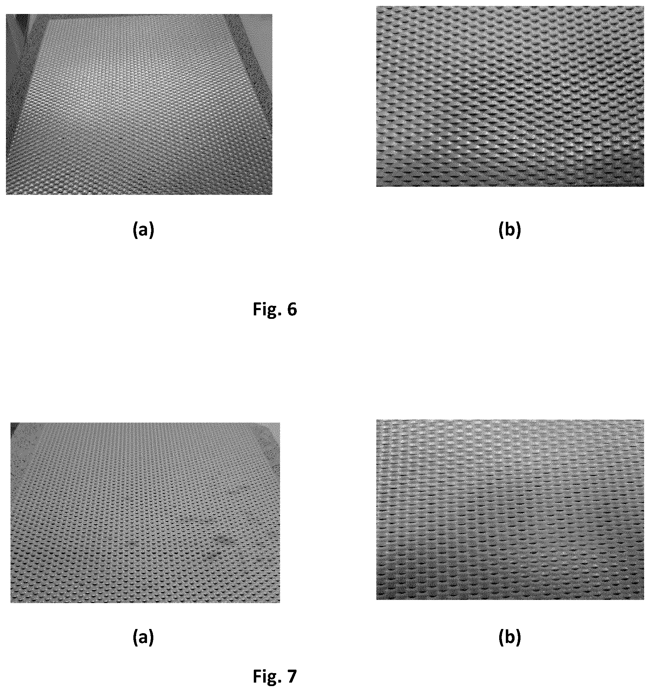

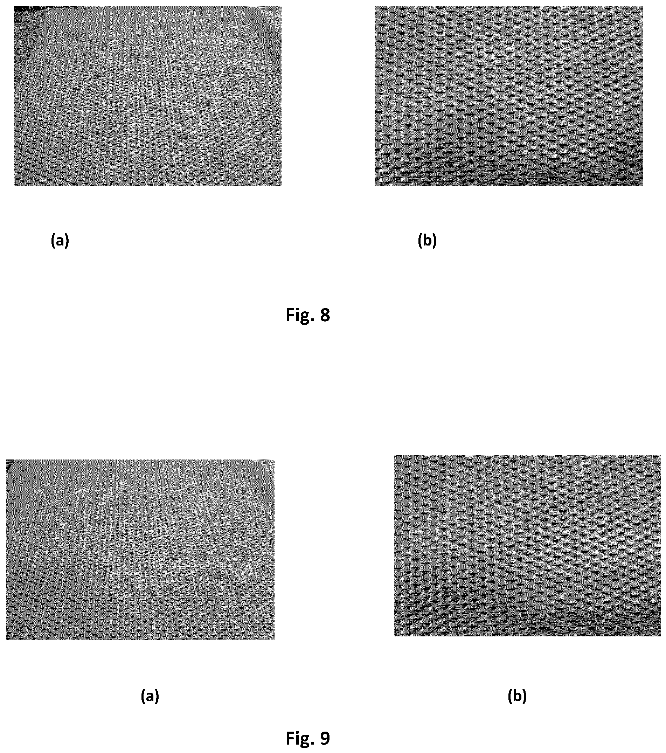

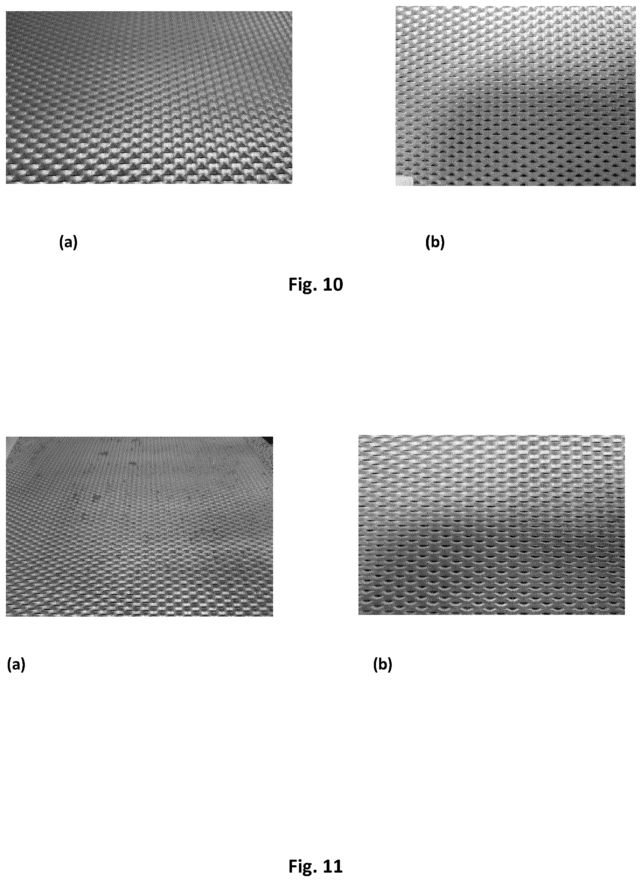

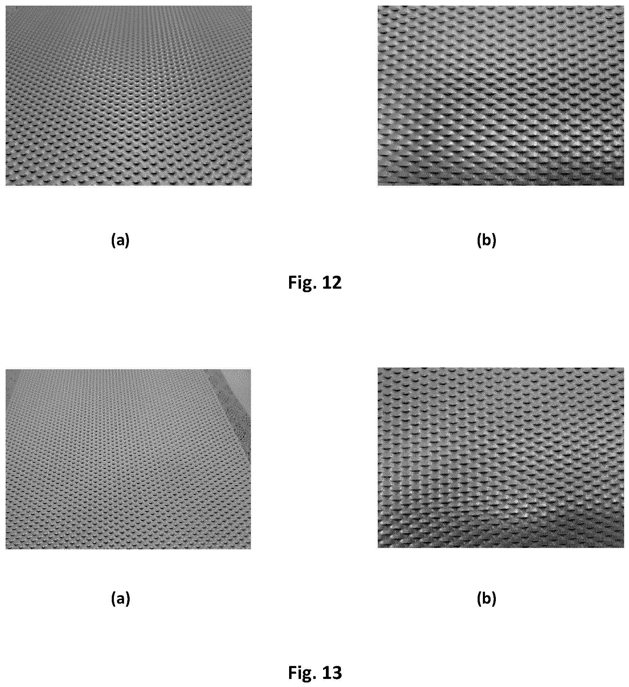

The Figures 6 to 13 are photographic representations of the coatings applied to 30 x 30 cm filters.

The term "comprises" or "comprising" whenever used in this document is intended to indicate the presence of stated features, elements, integers, steps and components, but not to preclude the presence or addition of one or more other features, elements, integers, steps and components, or groups thereof.

The present invention is not, naturally, in no way restricted to the embodiments described herein and a person with ordinary skill in the art may foresee many possibilities of modifications thereof and of replacing technical features by equivalent ones, depending on the requirements of each situation, as defined in the appended claims.

The following claims define additional embodiments of the present description.

## Claims

1. Air filter with photocatalytic coating for the inactivation of viruses, comprising a calcined mixture of nanoparticles of copper with titanium dioxide or with zinc oxide, wherein the amount of copper is 0.3 - 30% (wt/wt) relatively to the total weight of the coating.

2. Air filter according to the previous claim, wherein the photocatalytic coating comprises a mixture of copper with titanium dioxide, wherein the amount of copper is 0.3 - 30 % (wt/wt) relatively to the coating.

3. Air filter according to the previous claim, wherein the photocatalytic coating is a mixture of copper with zinc oxide, wherein the amount of copper is 0.3 - 30 % (wt/wt) relatively to the coating.

4. Air filter according to any of the previous claims, wherein the calcination temperature is 100 - 400 °C, more particularly 100 - 200°C, even more particularly approximately 100°C.

5. Air filter according to any of the previous claims, wherein the amount of copper is 0.5 - 15 % (wt/wt).

6. Air filter according to any of the previous claims, wherein the amount of copper is 0.5%; 1%; 3%; 6%; 10%; 12% or 15 % (wt/wt).

7. Air filter according to any of the previous claims, wherein the nanoparticles are obtainable by wet impregnation.

8. Air filter according to any of the previous claims, wherein the nanoparticles are obtainable by incipient impregnation.

9. Air filter according to any of the previous claims, wherein the air filter has a metal mesh, in particular, a metal mesh in aluminium.

10. Manufacturing method of the air filter of any of the previous claims, comprising the steps of:
providing a metal filter mesh;
obtaining copper nanoparticles impregnated with titanium dioxide or with zinc oxide;
obtaining a suspension by mixing the titanium dioxide or zinc oxide copper nanoparticles with a silane;
coating the metal mesh with the suspension obtained in the previous step;
curing the coated mesh at a temperature between 60 - 90 °C, preferably at 80 °C, for a period of about 20 - 28 hours, preferably 24 hours.

11. Method according to the previous claim, wherein when obtaining the nanoparticles, the copper is impregnated into titanium dioxide or zinc oxide by wet or incipient impregnation.

12. Method according to any of claims 9-10, wherein the obtention of nanoparticles comprises an additional step of thermal treatment at 100 - 400°C.

13. Method according to any of claims 9-10, wherein the amount of silane in the suspension is 90 % (v/v).

14. Method according to any of the previous claims, wherein the metal mesh is coated by spray technique.
